# DEMANDE DE BREVET EUROPEEN

(11) **EP 1 285 988 A1**
(43) Date de publication de la demande: **26.02.2003**
(21) Numéro de dépôt: 02292035.9
(22) Date de dépôt: 13.08.2002
(51) Int. Cl.: D06M 15/03, D06M 23/08, A61L 15/46

(54) **Sustrat non tissé pouvant dégager un arôme et/ou une saveur**

(30) Priorité: 21.08.2001 FR 0110954
(71) Demandeur: Tharreau Industries, 49120 Chemille (FR)
(72) Inventeur: Tharreau, Michel, 49000 Angers (FR)
(74) Mandataire: Cabinet Hirsch

(57) **Abrégé**

L'invention concerne un substrat non tissé pouvant dégager un arôme et/ou une saveur, notamment, par humidification.

Ce substrat non tissé est susceptible d'être obtenu par un procédé comprenant une étape de fixation thermique de particules de cyclodextrine sur le non-tissé.

Il peut être utilisé en particulier pour confectionner des compresses dentaires ou médicales.

L'invention concerne également un procédé de fabrication de ce substrat.

## Description

La présente invention concerne un substrat non tissé pouvant dégager un arôme et/ou une saveur, notamment, par humidification, qui peut être utilisé en particulier pour confectionner des compresses dentaires ou médicales.

Il est connu d'utiliser des cyclodextrines dans un non tissé pour améliorer les propriétés d'adsorption de ce dernier.

Ainsi, la demande de brevet français n° 2 789 704 propose un procédé de traitement d'une fibre ou d'un matériau tel qu'un non tissé, comprenant les opérations successives suivantes :
a) application d'un mélange solide de cyclodextrine(s) et/ou de dérivé(s) de cyclodextrine(s), d'au moins un acide poly(carboxylique) et éventuellement d'un catalyseur ;
b) chauffage à une température comprise entre 150°C et 220°C ;
c) lavage à l'eau ; et
d) séchage.

Il est également connu d'utiliser des complexes de cyclodextrine et de parfum pour traiter des tissus en vue de retarder la diffusion du parfum et/ou d'en contrôler la diffusion lorsque le tissu est humidifié.

Ainsi, la demande de brevet européen n° 392 607 a trait à un ensemble constitué :
- d'une composition de conditionnement de tissu, comprenant un agent adoucissant, une quantité efficace de complexe parfum/cyclodextrine et un agent de contrôle de la viscosité à base d'argile ; et
- de moyens de distribution pour distribuer une quantité efficace de composition de conditionnement de tissu sur le tissu dans une sécheuse fonctionnant à des températures de séchage automatique, par exemple, de 35 à 115°C.

Enfin, la demande internationale n° WO 99/21532 a pour objet un produit de conditionnement et de rinçage à usage personnel et unique, qui dépose sur la peau ou les cheveux des matériaux que l'on peut éliminer par rinçage et qui présente des propriétés de libération d'une fragrance satisfaisantes, ce produit comprenant :
(A) un substrat insoluble dans l'eau,
(B) au moins un tensio-actif moussant ajouté sur le substrat ou imprégnant le substrat,
(C) de 0,015% à 15% en poids du substrat insoluble, d'un complexe libérant la fragrance, ajouté sur le substrat ou imprégnant le substrat, ce complexe comprenant
   (i) de 10 à 90% en poids du complexe, d'un support poreux d'un composé libérant la fragrance, ce support pouvant être une cyclodextrine, et
   (ii) de 1 à 90% en poids du complexe, du composé libérant la fragrance, ce composé imprégnant le support,
ce produit étant essentiellement sec avant son usage.

Aucun de ces documents ne décrit ni ne suggère un substrat non tissé pouvant être utilisé dans le domaine dentaire, médical ou chirurgical.

L'invention a donc pour objet un substrat non tissé pouvant dégager un arôme et/ou une saveur et susceptible d'être obtenu par un procédé comprenant une étape de fixation thermique de particules de cyclodextrine sur le non-tissé.

Un tel substrat non tissé a l'avantage de pouvoir dégager un arôme et/ou une saveur par humidification ultérieure, au contact de l'eau ou, en cas d'usage bucco-dentaire, au contact de la salive.

Selon un premier aspect de l'invention, le non-tissé est fabriqué selon un premier procédé de fabrication, dit « procédé par immersion ». Ce procédé comprend les étapes suivantes :
a) immersion du substrat non tissé dans un bain aqueux, ou enduction avec une pâte ou une mousse, ledit bain aqueux ou ladite pâte ou mousse comprenant au moins des particules de cyclodextrine en suspension, ces particules contenant au moins une substance dégageant un arôme et/ou une saveur;
b) sortie du substrat non tissé du bain ou de l'enduction;
c) laminage du substrat non tissé ;
d) fixation thermique des particules de cyclodextrine.

Selon un second aspect de l'invention, le non-tissé est fabriqué selon un second procédé de fabrication, dit « procédé par enduction ». Ce procédé comprend les étapes suivantes :
a) enduction avec une poudre comprenant au moins des particules de cyclodextrine contenant au moins une substance dégageant un arôme et/ou une saveur;
b) pulvérisation d'eau sur le substrat enduit ;
c) fixation thermique des particules de cyclodextrine.

L'invention concerne également ces premier et second procédés.

D'autres caractéristiques et avantages de l'invention vont maintenant être décrits en détail dans l'exposé qui suit.

### EXPOSE DETAILLE DE L'INVENTION

Selon l'invention, le substrat non tissé de départ peut être constitué de matériaux choisis parmi les matériaux naturels ou synthétiques.

Il est en général constitué de fibres synthétiques ou naturelles ou d'un mélange de ces fibres.

Comme fibres naturelles, on peut citer par exemple, les fibres de soie, de kératine, de cellulose et leurs mélanges.

Comme fibres synthétiques, on peut citer par exemple, les fibres d'acétate, d'acrylique, d'ester de cellulose, de polyamide, de polyester, de polyoléfine, d'alcool polyvinylique, de rayonne, de mousse de polyuréthane et leurs mélanges.

Le substrat non tissé de départ est immergé dans un bain aqueux comprenant au moins des particules de cyclodextrine. Il peut également être enduit avec une poudre ou une pâte contenant des particules de cyclodextrine.

Comme cyclodextrine, on peut utiliser toute cyclodextrine ou tout dérivé de cyclodextrine ou tout mélange de cyclodextrines et/ou de dérivés de cyclodextrines, qui puisse former des complexes par inclusion d'une ou plusieurs substances dégageant un arôme et/ou une saveur.

On peut donc citer les cyclodextrines telles que les cyclodextrines non substituées contenant de 6 à 12 unités glucose, en particulier les cyclodextrines alpha, bêta et gamma et/ou leurs dérivés.

Des dérivés de cyclodextrines sont décrits par exemple dans les brevets américains n° 3 426 011, 3 453 257, 3 453 258, 3 453 529, 3 453 260, 3 459 731, 3 553 191, 3 565 887, 4 535 152, 4 616 008, 4 638 058, 4 746 734 et 4 678 598.

Les cyclodextrines individuelles peuvent aussi être liées les unes aux autres en utilisant des agents multifonctionnels pour former des oligomères, des polymères, des copolymères, etc.

Il est également possible d'utiliser des mélanges de cyclodextrines en vue de former des mélanges de complexes. De tels mélanges peuvent fournir des profils d'arômes et/ou de saveurs, en piégeant plusieurs substances dégageant une saveur et/ou un arôme et/ou en empêchant la formation de cristaux plus grands de tels complexes. Des mélanges de cyclodextrines peuvent être obtenus selon les enseignements des brevets américains n° 3 425 910, 3 812 011, 4 317 881, 4 418 144 et 4 738 923.

De préférence, on utilise essentiellement une cyclodextrine telle que les cyclodextrines alpha, bêta et/ou gamma et tout spécialement une cyclodextrine bêta.

Selon l'invention, les particules de cyclodextrine contiennent au moins une substance dégageant un arôme et/ou une saveur.

La substance dégageant un arôme et/ou une saveur peut être choisie parmi les parfums et les agents de refroidissement.

Par «agents de refroidissement », on entend dans le présent exposé les composés qui agissent sur les extrémités nerveuses provoquant les sensations de chaud ou de froid. On peut citer comme agents de refroidissement en particulier, le menthol, ses dérivés et les mélanges de ces composés.

En général, la substance dégageant un arôme et/ou une saveur est contenue dans les particules de cyclodextrine par la formation d'un complexe entre la cyclodextrine et la ou les substance(s) dégageant un arôme et/ou une saveur.

La formation d'un tel complexe s'effectue de manière connue de l'homme du métier. Habituellement, le complexe est formé par mise en contact de la cyclodextrine et de la substance dégageant un arôme et/ou une saveur sous forme de solutions ou de suspension dans des solvants appropriés, de préférence, l'eau ou en broyant les composés dans un minimum de solvant.

La formation du complexe peut avoir lieu comme étape préalable au procédé de fabrication du substrat non tissé selon l'invention.

Une fois l'immersion du substrat non tissé dans le bain aqueux de cyclodextrines réalisée, on sort le substrat et on le lamine entre deux cylindres, ce qui permet de contrôler et de réguler la quantité de suspension de cyclodextrine emportée et d'éliminer les excès de suspension.

Le substrat non tissé selon l'invention obtenu de cette manière possède donc la propriété intéressante de dégager ultérieurement, par exemple par humidification, un arôme et/ou une saveur.

L'humidification ultérieure peut avoir lieu par contact avec de l'eau.

Elle peut aussi avoir lieu au contact de la salive. Ainsi, lorsque ses constituants sont tous du type alimentaire, le non tissé selon l'invention peut trouver une application comme compresse médicale à usage bucco-dentaire.

### Exemples

Les exemples suivants illustrent la présente invention sans toutefois en limiter la portée.

### Exemple 1

En mettant en oeuvre le procédé selon l'invention (par immersion), on a fabriqué un substrat non tissé à partir duquel on a réalisé une compresse dentaire non tissée au goût mentholé.

### Exemple 2

Un échantillon de soupe alimentaire a été appliqué sur un substrat non tissé selon l'invention obtenu par le procédé (par enduction) selon l'invention.

## Revendications

1. Substrat non tissé pouvant dégager un arôme et/ou une saveur et susceptible d'être obtenu par un procédé comprenant une étape de fixation thermique de particules de cyclodextrine sur le non-tissé.

2. Substrat non tissé selon la revendication 1, susceptible d'être obtenu par un procédé comprenant les étapes suivantes :
a) immersion du substrat non tissé dans un bain aqueux, ou enduction avec une pâte ou une mousse, ledit bain aqueux ou ladite pâte ou mousse comprenant au moins des particules de cyclodextrine en suspension, ces particules contenant au moins une substance dégageant un arôme et/ou une saveur;
b) sortie du substrat non tissé du bain ou arrêt de l'enduction;
c) laminage du substrat non tissé ;
d) fixation thermique des particules de cyclodextrine.

3. Substrat non tissé selon la revendication 1, susceptible d'être obtenu par un procédé comprenant les étapes suivantes :
a) enduction avec une poudre comprenant au moins des particules de cyclodextrine contenant au moins une substance dégageant un arôme et/ou une saveur;
b) pulvérisation d'eau sur le substrat enduit ;
c) fixation thermique des particules de cyclodextrine.

4. Substrat non tissé selon l'une des revendications 1 à 3, dans lequel la cyclodextrine est choisie dans le groupe constitué par les cyclodextrines alpha, bêta et/ou gamma et leurs mélanges et est de préférence une cyclodextrine bêta.

5. Substrat non tissé selon l'une des revendications 1 à 4, dans lequel la substance dégageant un arôme et/ou une saveur est choisie dans le groupe constitué par le menthol, ses dérivés et les mélanges de ces composés.

6. Procédé de fabrication d'un substrat non tissé selon la revendication 1, comprenant les étapes suivantes :
a) immersion du substrat non tissé dans un bain aqueux, ou enduction avec une pâte ou une mousse, ledit bain aqueux ou ladite pâte ou mousse comprenant au moins des particules de cyclodextrine en suspension, ces particules contenant au moins une substance dégageant un arôme et/ou une saveur;
b) sortie du substrat non tissé du bain ou de l'enduction;
c) laminage du substrat non tissé ;
d) fixation thermique des particules de cyclodextrine.

7. Procédé de fabrication d'un substrat non tissé selon la revendication 1, comprenant les étapes suivantes :
a) enduction avec une poudre comprenant au moins des particules de cyclodextrine contenant au moins une substance dégageant un arôme et/ou une saveur;
b) pulvérisation d'eau sur le substrat enduit ;
c) fixation thermique des particules de cyclodextrine.

8. Procédé selon la revendication 6 ou la revendication 7, comprenant une étape préalable de formation d'un complexe entre les particules de cyclodextrine et au moins une substance dégageant un arôme et/ou une saveur.
